(19) Europäisches Patentamt
European Patent Office
Office européen des brevets

(11) Publication number: **0 072 142**
**B1**

(12) # EUROPEAN PATENT SPECIFICATION

(45) Date of publication of patent specification: **16.01.91**

(51) Int. Cl.$^5$: **C 01 F 7/00,** A 61 K 7/00,
A 61 K 9/06, A 61 K 33/08

(21) Application number: **82303962.3**

(22) Date of filing: **27.07.82**

(54) **Preparation of co-dried aluminium oxyhydroxides.**

(30) Priority: **03.08.81 US 289687**

(43) Date of publication of application:
**16.02.83 Bulletin 83/07**

(45) Publication of the grant of the patent:
**16.01.91 Bulletin 91/03**

(84) Designated Contracting States:
**DE FR GB**

(56) References cited:
**DE-A-2 163 036**
**US-A-3 599 150**
**US-A-4 115 553**

**Journal of the Applied Chemistry of the U.S.S.R.(1976), Vol. 49, No. 9, pp. 1009-1911**

(73) Proprietor: **REHEIS, INC.**
**235 Snyder Avenue**
**Berkeley Heights New Jersey 07922 (US)**

(72) Inventor: **Rubino, Andrew M.**
**25 Hickory Place Apartment G24**
**Chatham New Jersey 07928 (US)**
Inventor: **Siciliano, Arthur**
**14 Avondale Road**
**Denville New Jersey 07834 (US)**

(74) Representative: **Spencer, Graham Easdale et al**
**A.A. Thornton & CO Northumberland House**
**303-306, High Holborn**
**London WC1V 7LE (GB)**

**EP 0 072 142 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention is concerned with a process for the preparation of co-dried aluminium oxyhydroxides which are useful as adsorptive, protein-binding agents and with compositions comprising such aluminium oxyhydroxides for topical application to the skin.

It is well known to those skilled in the art that aluminium hydroxide gels are irreversibly degraded upon drying. The dehydration processing causes the loss of "water-of-constitution", among other entities, resulting in the destruction of most of the positive attributes characteristic of such gels, which properties serve as the very basis of their use in many applications. Illustrative degradative changes which can result are the following:

(1) alteration of the physiochemical structure of the products affecting their surfaces as well as their chemical reactivities;

(2) loss of adsorbent capacity;

(3) impairment of suspendability;

(4) increased density;

(5) increased particle size; and

(6) reduced viscosity.

The degradation of aluminium hydroxide gels upon dilution with water is discussed in "A Vehicle For Stabilizing Aluminum Hydroxide Gel", by Kerkhof, N. J.; Hem, S. L. and White, J. L., Journal of Pharmaceutical Sciences, 75, Vol. 64, No. 12, pp. 2030—2032, Dec. 1975.

U.S. Patents 4,112,072 and 4,115,553 are directed to producing dried antacids. These patents describe the co-drying of certain basic reactive aluminium bicarbonate-carbonate compounds with polyols to form novel antacid tablets.

U.S. 3,599,150 concerns a freeze stable suspension of an aluminium hydroxide gel. The suspension has an alum-precipitated aqueous extract or aqueous pyridine extract of an antibody producing antigenic substance absorbed thereon in an aqueous solution containing a water-soluble non-toxic polyhydric alcohol and water-soluble non-toxic polymers. This patent, however, is not concerned with co-drying of the aluminium hydroxide gel.

Zhurnal Prikladnoi Khimii (Journal of Applied Chemistry of the USSR), Vol, 49, No. 9, pp 1909—1911, September 1976, described the influence of organic substances on the formation of the porous structure of active alumina. For this purpose, small amounts of various organic substances, such as gelatin, polyoxyethylene, organic acids, etc., are introduced, in the form of aqueous solutions, into aluminium hydroxide precipitates which are plasticized with nitric acid. The porosity of the aluminium hydroxide particles after this treatment was then determined.

Non-reactive aluminium oxyhydroxides in the wet state, i.e., suspended in water, are known to be good adsorbers. For example, such suspensions are used in vaccines as toxin adsorbers (an adsorbent is a material that attracts and holds to its surface a gas, liquid or solid). Heretofore, these gels were useful only in the wet state. It was recognized that it was uneconomical to "ship large quantities of water around" with the aluminium hydroxide and that it would be advantageous to concentrate the aluminium hydroxide and resuspend it when needed. Attempts to dry such suspensions in order to concentrate them have heretofore not met with success due to the degradation of aluminium hydroxide upon drying as discussed above.

We have now found that by using certain additives which are capable of providing coordinating moieties with aluminium, aqueous suspensions of aluminium oxyhydroxide can be dried to give an adsorptive, protein-binding product and that such a product which is also resuspendable in water can be obtained by acidifying the aluminium oxyhydroxide-additive mixture.

According to the present invention, there is provided a method for the preparation of adsorptive, protein-binding aluminium oxyhydroxide, which comprises mixing an aqueous suspension of a carbonate-free, highly hydrated, aluminium oxyhydroxide gel which is relatively inert towards weak alkalis and acids, with a water-soluble additive which is a polyhydric alcohol, sugar, amino acid, polysaccharide or polyfunctional nitrogen-containing compound and which is capable of providing coordinating moieties with aluminium, and drying the mixture to form solid particles.

The aluminium oxyhydroxide used as starting material in the process according to the invention may be any inert aluminium hydroxide in which only the aluminium and the hydroxy and/or oxy entities are present. These compounds are distinguished from "reactive-grade aluminium hydroxide" which contains carbonate/bicarbonate. Such reactive compounds which find wide use as, inter alia, antacids are distinguished from inert aluminium hydroxide by simple carbon dioxide evolution analysis.

The additive mixed with the aluminium oxyhydroxide must be capable of furnishing coordinating moieties with aluminium and must be soluble in water. If the product of the co-drying method of the present invention is to be utilized as an agent in a skin composition, the additive must further be topically acceptable, i.e. safe for use on the skin.

Suitable polyhydric alcohols include, for example, glycerol, sorbitol, mannitol, polyethylene glycol (Carbowax), polypropylene glycol, butylene glycol and xylitol.

Suitable sugars include, for example, lactose, sucrose and glucose.

Suitable polyfunctional nitrogen-containing compounds include, for example, amino acids, such as L-glutamic acid, glycine and beta-alanine, and urea. If an amino acid is used in a suitable strength, i.e.

concentration, it can act both as an additive and as the acidifier when a re-suspendable product is desired.

Suitable polysaccharides include, for example, corn starch, "VEEGUM", "CERULOSE", "CARBOPOLS" and "CELLOSIZE" (Trade marks).

The weight ratio of aluminium oxyhydroxide, calculated as aluminium oxide, to additive present in the suspension prior to drying is suitably from 10:1 to 3:2, and preferably from 6:1 to 3:2. Based on the product, after drying, the additive can amount to from 5 to 30% by weight of the total product. The point of determination of the amount of additive, either before or after drying, is not *per se* critical and only has significance when volatile additives are utilized. A volatile additive will dissipate to some degree during drying depending on its volatility and the drying temperature. The weight ratios of additive to aluminium oxide as given above assume no loss of additive during drying. It would be a simple matter for one practising this invention to calculate the estimated loss of additive due to evaporation and then add extra additive in anticipation of such loss. For a non-volatile additive, such as corn starch, this procedure is, of course, unnecessary.

The mixture of additive and aluminium oxyhydroxide compound is subjected to co-drying at appropriate temperatures. High drying temperatures should not, however, be used since there is a tendency to break bonds at high temperatures. It is advantageous to drive off as much moisture as possible without over-drying the mixture. It is preferred to dry the mixture to attain from 55 to 65% by weight aluminium oxyhydroxide, calculated as aluminium oxide.

Any convenient drying method can be employed, such as spray drying, vacuum drying, and hot air circulating ovens. The type of drying used will generally determine the particle size of the resultant solids. It is preferred in many instances to form a fine powder. Such powder can, for example, be produced using a spray drying technique or other drying techniques which produce larger particles which are subsequently comminuted by various grinding techniques known in the art.

Although it is usually desired to minimize the amount of additive, such amount is directly proportional to the protein-binding capacity of the product. Protein binding is enhanced by increasing the surface area of the aluminium-containing compound particles, i.e., fine particle size. The suspendability of the dried products of this invention is inversely proportional to the pH of their respective suspensions.

The more concentrated the product, the less expensive the shipping costs due to elimination of unnecessary water being carried along with the useful product. Thus it is advantageous to obtain a product that is readily re-suspendable in water.

To obtain a protein-binding, adsorptive agent that is re-suspendable, acid must be utilized. Acidification can be effected either prior to co-drying or after co-drying. It is preferred to acidify prior to co-drying in order to produce a concentrated re-suspendable solution. In this case, the aluminium oxyhydroxide in aqueous suspension is acidified to give a pH for the suspension of from 4 to 6.5, preferably from 5 to 6. The acidified suspension is then mixed with the additive and co-dried to the desired degree. Alternatively, the aluminium oxyhydroxide aqueous suspension can be mixed with additive and then acidified to the pH range as given above prior to co-drying. The acidification can be effected with any moderately strong acid, for example hydrochloric acid, nitric acid or sulphuric acid. Acidification can also be carried out with compounds which yield acids upon hydrolysis, such as $AlCl_3$.

Acidification can also be effected after co-drying. In this case, the co-dried product is mixed with water to form a suspension and the latter is acidified to the pH range as given above.

The adsorptive, protein-binding aluminium oxyhydroxide product produced by the method of the present invention can be employed in topical skin compositions. It can be used alone or in combination with other ingredients to form compositions which can be used as sun screens, wrinkle fillers, acne medications, poison ivy remedies, burn remedies, anti-fungicides, antiseptics, analgesics, cosmetics, for example, lipsticks, and skin creams. The other ingredients used in conjunction with the product of the present invention to form topical skin compositions include emulsifiers, such as a combination of glycerol monostearate, triethanolamine and stearic acid; thickeners, such as stearic acid; emollients, such as isopropyl myristate; and humectants, such as glycerine. Typical formulations are, for example, as follows:

Co-dried powder produced in accordance with the present invention—approximate analysis

| | |
|---|---|
| wt.% $Al_2O_3$ | 60.10 |
| wt.% Glycerine | 17.30 |
| wt.% Chloride | 3.40 |
| wt.% Na | 0.45 |
| wt.% $SO_4$ | 0.71 |
| wt.% Water | 19.04 (some water may exist as $OH^-$) |
| pH (4% Suspension) | 5.1 |
| Bulk density | 0.77 g/cc |
| Protein binding capacity | 1.50 mg/mg $Al_2O_3$ at pH 5.5 |

Ointment

| | |
|---|---|
| $Al_2O_3$—Glycerine powder as given above | 3.2 wt.% |
| Mineral oil | 15.0 wt.% |
| Petrolatum | 76.8 wt.% |
| Polyoxyethylene 4 sorbitan monostearate ("TWEEN 61"; Trade Mark) | 5.0 wt.% |

Powder

| | |
|---|---|
| $Al_2O_3$—Glycerine powder as given above | 3.2 wt.% |
| Talc | 93.8 wt.% |
| Magnesium stearate | 3.0 wt.% |

Creams

| | wt.% | wt.% |
|---|---|---|
| $Al_2O_3$—Glycerine powder as given above | 5.0 | 5.0 |
| "ARLACEL 165" (glycerol monostearate and polyoxyethylene sterate; Trade Mark) | 3.5 | 3.5 |
| Mineral oil | 5.0 | 3.0 |
| Isopropyl myristate | 3.0 | 6.0 |
| Propylene glycol | 2.0 | 1.0 |
| Cetyl alcohol | 1.5 | 4.0 |
| Methyl paraben | 0.2 | 0.2 |
| Propyl paraben | 0.05 | 0.05 |
| "MYRJ 52" (polyoxyethylene 40 stearate; Trade Mark) | — | 0.5 |
| Titanium dioxide | — | 0.2 |
| Deionized water | 79.75 | 76.55 |

The present invention gives an aluminium oxyhydroxide agent having the following attributes:

(1) oil absorption (an absorbent is a material that "sucks up" or incorporates liquids, gases, etc);
(2) thixotropic;
(3) protein-binding;
(4) resistant to being washed away;
(5) aesthetically pleasant;
(6) stable;
(7) non-tacky;
(8) non-greasy;
(9) non-irritating;
(10) non-toxic;
(11) compatible with oxidizing agents, for example, hydroperoxide and benzoperoxide;
(12) having a broad pH range; and
(13) re-suspendable (when acidified).

In order that the invention may be more fully understood, the following examples are given by way of illustration only:

Examples 1—10

Effect of glycerol levels on re-suspendability of co-dried products

Examples 1—5 (without HCl)

Five different $Al_2O_3$/glycerol weight ratio levels were utilized for examintion of re-suspendability; 3/0, 6/1, 3/1, 2/1, 1.5/1. To each bath of $Al_2O_3$, glycerol was added according to the formula:

$$\frac{(\text{wt. } Al_2O_3)\ (\%Al_2O_3 \text{ gel})\ (0.01)}{\text{Ratio } Al_2O_3/\text{glycerol desired}}$$

The source for $Al_2O_3$ used throughout all the examples herein was Reheis Chemical Company "F-5000" gel. "F-5000" is a carbonate-free, highly hydrated, aluminium oxyhydroxide gel which is relatively inert towards weak alkalis and acids.

5500 grams of 9.6 wt.% $Al_2O_3$ were added to an 18 liter Nalgene polypropylene vessel. Ten liters of well water were added to the gel and the mixture was homogenized with a Premier dispersator for two hours. From this slurry, five aliquots of 3000 grams each were taken, and each was placed in an 8 liter Nalgene polypropylene vessel.

Example 1

To the first aliquot was added 17.1 grams of glycerol according to the above formula. The slurry was agitated for an hour and screened through a #40 (0.42 mm) sieve before spray drying. Before the slurry was spray dried, two liters of well water were added to the slurry to improve pumpability. The slurry was spray dried at 120—125°C in a 3 foot (0.91 m) circular Bowen spray dryer employing a two-fluid nozzle head. Air pressure was set at 80 psig (5.6 Kg/cm$^2$) and feed rate at 100 ml/min. This procedure yielded about 150 grams of finely divided, free-flowing, white powder with a pre-spray dry $Al_2O_3$ to glycerol rate of 6/1 (see Table 1).

To demonstrate the resuspendability of the $Al_2O_3$-glycerol co-dried system with HCl, 2 grams of the above powder was added to 25 grams deionized water in a 4"×1" glass vial. With its cap in place, the vial was shaken ten times and three drops (approximately 0.14 grams) of 31% HCl was added to the vial by a medicine dropper. The vial was again shaken ten times. The vial approximated 4 wt% $Al_2O_3$ (Example 1A). Resuspendability of an 8% system was also tested using 4 grams of powder and 5 drops (approximately 0.24 grams) of 31% HCl in 25 grams deionized water (Example 1B).

After standing overnight, the pH's of the vials were measured with a Beckman SS-3 pH meter standardized at pH 5 and pH 7.

Example 2

This example is identical to the above Example 1 with the exception that 34.1 grams of glycerol were added to the slurry for a projected 3/1 ratio of $Al_2O_3$ to glycerol. Spray drying parameters and resuspendability techniques for 4% and 8% resuspensions were identical to Example 1 and for succeeding Examples 3—5.

Example 3

In this example 51.2 grams of glycerol was used to achieve a 2/1 ratio of $Al_2O_3$ to glycerol.

Example 4

In this example 68.2 grams of glycerol was used with the resultant slurry having a 3/2 ratio of $Al_2O_3$ to glycerol.

Example 5

No glycerol was used in this slurry which served as the 3/0 control.

The above spray dried slurries were analyzed for % $Al_2O_3$ and reported in Table I herein.

Resuspendability of the $Al_2O_3$-glycerol co-dried systems with HCl added on resuspension is shown in Table II herein.

Examples 6—10 (with HCl)

5000 grams of 9.6 wt.% $Al_2O_3$ were added to an 18 liter Nalgene polypropylene vessel. To this, three liters of well water were added. The pH of the slurry was then adjusted to 4.8 with 62 ml of 31% HCl under constant agitation with a Premier dispersator. The mixture was homogenized for two hours. From this slurry, five aliquots of 1800 grams each were taken and placed in an 8 liter Nalgene polypropylene vessel.

Example 6

To the first 1800 gram aliquot was added 18 grams of glycerol under constant agitation with the dispersator. The slurry was agitated one hour, and then screened through a #40 (0.42 mm) mesh sieve before spray drying (No extra well water was added to the slurry since it was pumpable). The spray drying parameters followed exactly those enumerated above in Example 1. 130 grams of finely divided, free-flowing powder resulted with a 6/1 pre-spray dry $Al_2O_3$ to glycerol ratio (see Table II).

Resuspendability of this system was achieved by adding 2 grams of the powder to 25 grams deionized water in a 4"×1" (10.2×2.54 cm) glass vial. The vial was shaken ten times and left to stand overnight. After a minimum of 24 hours, pH's and descriptions were recorded.

Example 7

This example was identical to Example 6 except 36 grams of glycerol was added to the slurry to yield a 3/1 ratio. Dilution, spray drying parameters and resuspendability techniques for 4% resuspensions were identical to Example 6 for this and the succeeding Examples 8 through 10.

Example 8

The third aliquot received 54 grams of glycerol for an $Al_2O_3$/glycerol ratio of 2/1.

Example 9

72 grams of glycerol were added to the fourth 1800 gram portion for a 3/2 ratio $Al_2O_3$ to glycerol.

Example 10

To the last aliquot of slurry, no glycerol was added.

The spray dried slurries in each of the above Examples 6—10 produced 130—140 grams of finely divided, free-flowing, white powder. Analyses of these powders is given in Table III herein.

Table IV given herein describes resuspendability of the $Al_2O_3$-glycerol-HCl system at various glycerol concentrations.

Examples 11—15

Effect of pH on resuspendability

In the following examples, the effect of pH on $Al_2O_3$-glycerol-HCl co-dried systems is demonstrated. Varying amounts of HCl was used prior to spray drying to achieve powders or different pH values.

To 6000 grams of 9.6% $Al_2O_3$ gel in an 18 liter Nalgene polypropylene vessel was added 3600 grams of well water and the resultant mixture was slurried with a Premier dispersator for two hours. This slurry was then split into five 1800 grams aliquots each and put into 8 liter Nalgene polypropylene vessels. Each of the five portions was acidified to a different extent as shown in Examples 11 through 15.

Example 11

To the first 1800 gram aliquot was added 36 grams glycerol and 15.7 ml 31% HCl under constant agitation from a dispersator. The pH was measured and found to be 4.3. 130 grams of finely divided, free-flowing, white powder resulted after spray drying at parameters mentioned in Example 1. Resuspension of this powder and the powders of Examples 12 through 15 were according to the procedures of Example 6.

Example 12

To this aliquot was added 36 grams of glycerol and 138 ml 31% HCl. After agitation, the pH was read as 4.8.

Example 13

36 grams of glycerol and 12.7 ml HCl were added to the third aliquot to give a final pH of 5.5. Also, one liter of well water was added to make the slurry pumpable.

Example 14

36 grams of glycerol, 8.3 ml 31% HCl and three liters of well water were added to this sample.

Example 15

36 grams of glycerol, 8.3 ml 31% HCl, three liters of well water, and no HCl were added to this sample before spray drying.

The results of the resuspendibility tests at different pH's are given in Table V herein.

Examples 16—20

Effect of concentration on resuspendability

Superior resuspendability of this invention over a wide range of concentrations are shown below using $Al_2O_3$-glycerol-HCl co-dried systems. Each of the examples outlined below (Examples 16 through 20) used powder synthesized in accordance with the procedures of Example 7. The results for Examples 16—20 are given in Table VI herein.

Example 16

One gram of Example 7 powder and 25 grams of deionized water were put into a 4"×1" (10.2×2.54 cm) glass vial and shaken ten times. This represented the 2% $Al_2O_3$ example. Except for powder quantities, all resuspensions were performed identically and followed the procedures given in Example 6.

Example 17

This example is a duplicate of Example 7.

Example 18

Three grams of powder gave approximately a 6% $Al_2O_3$ resuspension.

Example 19

Four grams of powder resulted in an 8% resuspension.

Example 20

Five grams of powder resuspended for a 10% $Al_2O_3$ level.

Examples 21—33

Order of component addition in relation to spray drying on resuspendability.

Resuspendability studies were performed on four different variations of co-dried powders: $Al_2O_3$

alone, $Al_2O_3$ co-dried with glycerol, $Al_2O_3$ co-dried with glycerol and HCl, and $Al_2O_3$ co-dried with HCl. To each of these powders glycerol, HCl, or both were added after spray drying. The resulting thirteen examples examined at 4% and 8% $Al_2O_3$.

5000 grams of 9.6 wt.% $Al_2O_3$ were added to an 18 liter Nalgene polypropylene vessel. Nine liters of well water was added to the gel and the mixture was homogenized with a Premier dispersator for two hours. This batch was then split into two unequal sized aliquots; one, 7625 grams and the other, 6120 grams. The larger portion was acidified to pH 4.8 with 27 ml of 31% HCl in an 8 liter Nalgene polypropylene vessel. The smaller portion received no HCl.

The acidified batch was split into two equal portions of 3800 grams, each put into an 8 liter Nalgene polypropylene vessel. To one of these 3800 gram batches was added 43.4 grams of glycerol. This batch was agitated for one hour. The other 3800 gram batch received no glycerol.

The unacidified half was also split into two aliquots of about 3060 grams each. Again, each was placed in an 8 liter Nalgene polypropylene vessel. One portion received 35.0 gram glycerol according to the formula given for Examples 1—5, and one portion received no glycerol. The regime for Examples 21—33 is given in Table VII herein; the results for Examples 21—33 are given in Tables VIII and IX herein.

The slurries were all spray dried according to Example 1, but 2 liters of well water had to be added to the unacidified slurries to make them pumpable.

Examples 21—26
Spray dried $Al_2O_3$ plus UnCo-dried adjuvants
Example 21A

To a 4″×1″ (10.2×2.54 cm) glass vial containing 25 grams of deionized water was added two grams of $Al_2O_3$ powder as prepared above. The vial was shaken ten times and then 4 drops of 31% HCl were added to the vial by a medicine dropper and shaken again for ten times. The vial was left overnight at which time a pH reading was taken and observations were made. This represented a 4% $Al_2O_3$ sample. Examples 22 through 26 follow the above procedure with the stated changes.

Example 21B

An 8% $Al_2O_3$ sample was also made up with 4 grams of powder in 25 grams of deionized water plus 5 drops HCl.

Example 22A

4% $Al_2O_3$ and 3 drops HCl.

Example 22B

8% $Al_3O_3$ and 4 drops HCl.

Example 23A

4% $Al_2O_3$, 3 drops glycerol (0.2 grams), and 4 drops HCl. Amount of glycerol to each vial was figured according to formula given in Examples 1—5.

Example 23B

8% $Al_2O_3$, 6 drops glycerol (0.4 grams), and 5 drops HCl.

Example 24A

4% $Al_2O_3$, 3 drops HCl, 3 drops glycerol.

Example 24B

8% $Al_2O_3$, 4 drops HCl, and 3 drops glycerol.

Example 25A

4% $Al_2O_3$ and 6 drops glycerol.

Example 25B

8% $Al_2O_3$ and 6 drops glycerol.

Example 26A

4% $Al_2O_3$; no additives.

Example 26B

8% $Al_2O_3$; no additives.

Examples 27—29

$Al_2O_3$-glycerol co-dried powder was used in Examples 27 to 29. These resuspensions were prepared according to the procedure in Example 21A.

Example 27A
4% $Al_2O_3$ and 3 drops HCl.

Example 27B
8% $Al_2O_3$ and 4 drops HCl.

Example 28A
4% $Al_2O_3$ and 4 drops HCl.

Example 28B
8% $Al_2O_3$ and 5 drops HCl.

Example 29A
4% $Al_2O_3$; no additives.

Example 29B
8% $Al_2O_3$; no additives.

Examples 30—31
$Al_2O_3$-Glycerol-HCl co-dried systems—no additives

$Al_2O_3$-Glycerol-HCl Co-Dried powder was used in Examples 30A and 30B. These resuspensions also followed the procedure given in Example 21A.

Example 30A
4% $Al_2O_3$

Example 30B
8% $Al_2O_3$

Example 31A
4% $Al_2O_3$ using powder prepared according to Example 11.

Example 31B
8% $Al_2O_3$ using powder prepared according to Example 11.

Examples 32—33
$Al_2O_3$-HCl co-dried systems

$Al_2O_3$-HCl Co-Dried powder was used in Examples 32 and 33. These resuspensions followed the procedure given in Example 21A.

Example 32A
4% $Al_2O_3$ and 3 drops glycerol.

Example 32B
8% $Al_2O_3$ and 6 drops glycerol.

Example 33A
4% $Al_2O_3$; no additives.

Example 33B
8% $Al_2O_3$; no additives.

Examples 34—50
Resuspendability of $Al_2O_3$-adjuvant co-dried systems

Examples 34—50 show the superior resuspendability of $Al_2O_3$ gel co-dried with a number of additives, thirteen of eighteen having a positive effect on resuspendability.

5500 grams of $Al_2O_3$ gel and ten liters of well water were slurried with a dispersator in an 18 liter reaction vessel for two hours. Four 4000 gram aliquots were taken from this main batch and each was mixed in four 8-liter reaction vessels. This diluted the gel from 9.6% $Al_2O_3$ to 3.4% $Al_2O_3$. All slurries possessed 3/1 $Al_2O_3$ to additive ratios. Analyses for the powders used in Examples 34—50 are given in Table X herein. The results for Examples 34—50 are given in Tables XI and XII herein.

Example 34A

To one of the 4000 gram aliquots was added 45.5 grams of sucrose according to the formula given for Examples 1—5. The slurry was mixed in a dispersator for one hour. Two liters of additional well water were added to make the gel pumpable. The slurry was then spray dried and resuspended at 4% according to Example 1. The following Examples 35 to 50 follow the above example except that in Examples 38 through 50, 3000 grams of slurry and 34.1 grams of additive were used. The additives utilized were as follows:

sucrose—sucrose USP
lactose—Fisher ACS α-lactose
sorbitol—ICI 70% sorbitol solution
polypropylene glycol
Carbowax 200—Union Carbide "CARBOWAX 200"; Grade 1, 2 propanediol
Carbowax 400—Union Carbide "CARBOWAX 400"
Carbowax 600—Union Carbide "CARBOWAX 600"
stearic acid—Baker certified stearic acid
citric acid—Pfizer citric acid USP
potassium citrate—Fisher certified potassium citrate
corn starch—Sargent-Welsh corn starch
Gelatin—Atlantic Gelatin edible 200B
glycine—Walker glycine USP
beta-alanine—Fisher beta-alanine purified grade
L-Glutamic acid—Fisher certified glutamic acid
Veegum—"VEEGUM" regular grade—R. T. Vanderbilt Inc.
Urea—Fisher ACS grade
PVP K-30—General Aniline & Film Corp. PVP (polyvinylpyrolidine) K-30 USP
PVP K-60—General Aniline & Film Corp. PVP (polyvinylpyrolidine)

Examples 51—71

Protein binding

A known excess of protein standard was mixed with the sample. The non-adsorbed protein was spectrometrically determined and subtracted from the total added protein.

Protein reagent

A protein reagent was prepared by pipetting 30 ml of deionized water into a 100 ml breaker. 1.596 grams of bovine albumin Fraction V powder, Armour Pharmaceutical Company, was added to the water. The resultant mixture was gently stirred to effect solution. The solution was then filtered through Whatman #4 (4.76 mm) filter paper and stored in a glass stoppered flask.

Preparation of standard curve

Protein reagent as prepared according to the above was pipetted in amounts of 0.5, 1.0, 1.5 and 2.0 ml respectively into four different 50 ml volumetric flasks and each flask was diluted to the mark with deionized water. The four standards were each well mixed. Each standard contained 0.25, 0.50, 0.75 and 1.00 mg. protein per/ml respectively. Absorbance of each standard was obtained at a wavelength of 280 nm using 10 mm silica cells on a Beckman Model 25 spectrophotometer using deionized water as a blank. A standard graph was prepared by plotting absorbance vs. concentration of each protein standard (mg. Protein/ml) on linear graph paper. The best possible straight line to fit most of the points and the origin was drawn.

Procedure

To at least three significant figures two and four grams of the $Al_2O_3$ samples were weighed into two separate 50 ml glass stoppered centrifuge tubes (concentration of each sample was approximately 40 mg and 80 mg $Al_2O_3$). A sufficient amount of deionized water was added from a burette so that grams of sample plus milliliters of water equaled 17.0 mls (if the density of the suspension was significantly different from one, corrections were made for the sample volume). The samples were shaken gently to disperse the suspension. 3.00 ml (150 mg of protein) of protein reagent (Bovine Albumin F. V, 5% Solution) solution was then added. The tubes were then mixed gently and placed on a Burrell wrist action shaker. The tubes were shaken gently at a setting of 2 for 15 minutes.

The tubes were centrifuged for 15 minutes at 2000 to 2500 RPM. The supernatant was filtered through a millipore HA (0.45 μm) membrane filter paper. 10 ml of clear filtrate was pipetted into a 100 ml volumetric flask and diluted to volume with deionized water. The absorbance for each sample at 280 nm wavelength was read using 10 mm silica cells, the model 25 spectrophotometer and deionized water as a blank. The concentration of each non-adsorbed protein sample was obtained in mg/ml at approximately 40 mg $Al_2O_3$ and 80.0 mg $Al_2O_3$ level from the standard graph.

Calculations

@ approximately 40 mg $Al_2O_3$ level,

$$\frac{\text{mg Protein adsorbed}}{\text{mg } Al_2O_3} = 150 - \frac{\text{mg/ml Protein from graph} \times 2 \times 100}{\text{mg. of } Al_2O_3 \text{ in suspension}}$$

and,

@ approximately 80 mg $Al_2O_3$ level,

$$\frac{\text{mg Protein adsorbed}}{\text{mg } Al_2O_3} = 150 - \frac{\text{mg/ml Protein from graph} \times 2 \times 100}{\text{mg of } Al_2O_3 \text{ in suspension}}$$

Results

The average of both the mg Protein/mg $Al_2O_3$ were taken at approximately 40 mg $Al_2O_3$ and 80 mg $Al_2O_3$ level and reported as mg Protein/mg $Al_2O_3$.

Using the above procedure, protein-binding capacities were determined for various co-dried compounds in Examples 51—71 and the results are given in Table XIII herein.

Since surface area plays a major role in the adsorption measured in this test, micronization might increase the values of the co-dried systems and not the $Al_2O_3$ dried without additives.

Examples 72—91

Protein binding

The same test procedure as in Examples 51—71 was utilized in Examples 72—91 except the pH was adjusted to 5.5. The glycerine control (100% glycerine) test was not applicable, since both it and the protein solution were soluble in mixed form.

A plot of protein binding capacity vs. % glycerine for co-dried $Al_2O_3$-glycerine without acid is given in the single Figure of the accompanying drawing (Example Nos. 72—75). The results for Examples 72—91 are given in Table XIV herein.

Examples 92—100

In Examples 92—100, co-dried systems of $Al_2O_3$-glycerine prepared in accordance with this invention were evaluated with and without the addition of acid (HCl). The results for Examples 92—100 are given in Table XV herein. It is evident from Table XV that re-suspendability was afforded to only those systems employing acid. The use of acid also increased the protein binding capacity of the co-dried systems.

TABLE I

| Example No. | %$Al_2O_3$ | $Al_2O_3$/Glycerol ratio in slurry |
|---|---|---|
| 1 | 61.4 | 6/1 |
| 2 | 60.1 | 3/1 |
| 3 | 58.6 | 2/1 |
| 4 | 57.9 | 3/2 |
| 5 | 64.47 | 3/0 |

TABLE II

| Example No. | pH Measured | $Al_2O_3$/Glycerol ratio in slurry | *Description |
|---|---|---|---|
| 1A | 4.7 | 6/1 | fluid gel |
| 1B | 6.0 | 6/1 | thick fluid gel |
| 2A | 5.1 | 3/1 | gel |
| 2B | 6.3 | 3/1 | gel |
| 3A | 4.7 | 2/1 | fluid gel |
| 3B | 6.3 | 2/1 | gel |
| 4A | 4.0 | 3/2 | fluid gel |
| 4B | 6.3 | 3/2 | gel |
| 5A | 4.7 | 3/1 | fluid gel |
| 5B | 6.2 | 3/0 | with 0.2 cm unsuspended powder |

*All resuspensions described at a minimum of 1 day, maximum of 30 days.

TABLE III

| Example No. | $\%Al_2O_3$ | %Glycerol | $Al_2O_3$/Glycerol ratio | |
|---|---|---|---|---|
| | | | Slurry | Powder |
| 6 | 62.18 | 10.5 | 6/1 | 5.9/1 |
| 7 | 59.20 | 16.5 | 3/1 | 3.6/1 |
| 8 | 56.70 | 19.7 | 2/1 | 2.9/1 |
| 9 | 55.77 | 22.9 | 3/2 | 4.9/2 |
| 10 | 64.47 | — | 3/0 | 3/0 |

TABLE IV

| Example No. | Measured pH | Description |
|---|---|---|
| 6 | 5.8 | gel |
| 7 | 5.8 | gel |
| 8 | 5.6 | gel |
| 9 | 5.7 | gel |
| 10 | 5.9 | 0.5 cm unsuspended powder in 6 cm supernatant |

TABLE V

| Example No. | Measured pH | $Al_2O_3$/Glycerol in slurry | Description |
|---|---|---|---|
| 11 | 5.5 | 3/1 | gel |
| 12 | 5.9 | 3/1 | gel |
| 13 | 6.4 | 3/1 | fluid gel |
| 14 | 7.2 | 3/1 | 0.5 cm unsuspended powder and 6 cm supernatant |
| 15 | 7.9 | 3/1 | same as Example 14 |

TABLE VI

| Example No. | Measured pH | Approx. % $Al_2O_3$ concentration in vial | Description |
|---|---|---|---|
| 16 | 5.7 | 2 | fluid gel |
| 17 | 5.7 | 4 | gel |
| 18 | 5.7 | 6 | gel |
| 19 | 5.8 | 8 | very thick gel |
| 20 | 5.7 | 10 | very thick gel |

TABLE VII

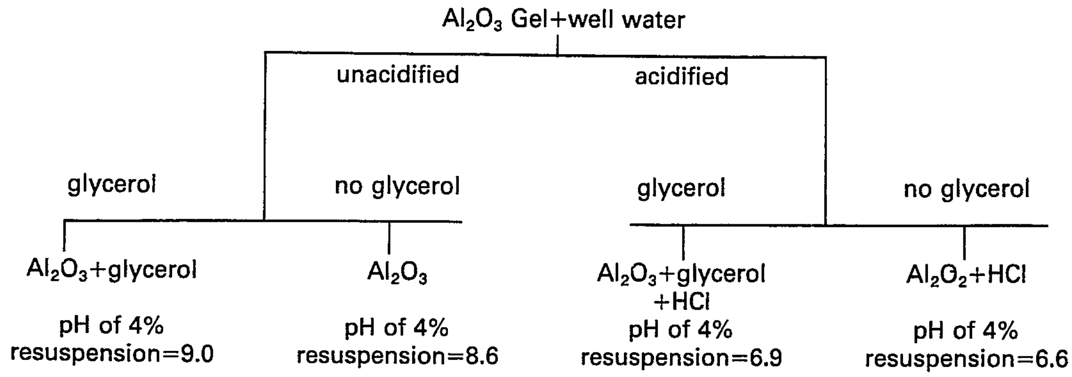


TABLE VIII

| Example No. | Measured pH | %$Al_2O_3$ | Resuspension | *Description |
|---|---|---|---|---|
| 21A | 4.6 | 4 | fair | 0.5 cm settled powder below hazy liquid |
| 21B | 5.0 | 8 | fair | 2 cm clear liquid above unsuspended powder |
| 22A | 5.5 | 4 | fair | 1 cm settled powder above hazy liquid |
| 22B | 6.2 | 8 | fair | 2 cm clear liquid above unsuspended powder |
| 23A | 4.2 | 4 | fair | 0.5 cm settled powder below hazy powder |
| 23B | 5.7 | 8 | good | thick suspension |
| 24A | 5.2 | 4 | fair | 1.5 cm settled powder below hazy liquid |
| 24B | 6.2 | 8 | fair | 1.5 cm clear liquid above settled powder |
| 25A | 8.4 | 4 | no | unsuspended powder |
| 25B | 8.5 | 8 | no | unsuspended powder |
| 26A | 8.5 | 4 | no | unsuspended powder |
| 26B | 8.4 | 8 | no | unsuspended powder |
| 27A | 5.2 | 4 | good | gel |
| 27B | 6.5 | 8 | good | very thick gel |
| 28A | 4.1 | 4 | good | fluid gel |
| 28B | 6.2 | 8 | good | very thick gel |
| 29A | 8.9 | 4 | no | unsuspended powder |
| 29B | 8.9 | 8 | no | unsuspended powder |
| 30A | 6.3 | 4 | no | unsuspended powder |
| 30B | 6.5 | 8 | no | unsuspended powder |
| 31A | 5.6 | 4 | good | gel |
| 31B | 5.8 | 8 | good | very thick gel |
| 32A | 6.3 | 4 | no | unsuspended powder |
| 32B | 6.5 | 8 | no | unsuspended powder |
| 33A | 6.3 | 4 | no | unsuspended powder |
| 33B | 6.5 | 8 | no | unsuspended powder |

*All descriptions made at minimum 1 day, maximum 30 days.

TABLE IX
Reevaluation of resuspendability taken
about six weeks later than those taken in Table VIII

| Example No. | %$Al_2O_3$ | Resuspension | *Description |
|---|---|---|---|
| 21A | 4 | good | gel |
| 21B | 8 | good | gel |
| 22A | 4 | fair | 1.5 cm clear liquid above 4 cm resuspended powder |
| 22B | 8 | fair | 0.5 cm clear liquid above 5 cm resuspended powder |
| 23A | 4 | good | gel |
| 23B | 8 | good | gel |
| 24A | 4 | good | fluid gel |
| 24B | 8 | fair | 1 cm clear liquid above 4.5 cm resuspended powder |
| 25A | 4 | no | unsuspended powder |
| 25B | 8 | no | unsuspended powder |
| 26A | 4 | no | unsuspended powder |
| 26B | 8 | no | unsuspended powder |
| 27A | 4 | good | gel |
| 27B | 8 | good | very thick gel |
| 28A | 4 | good | gel |
| 28B | 8 | good | very thick gel |
| 29A | 4 | no | unsuspended powder |
| 29B | 8 | no | unsuspended |
| 30A | 4 | fair | 1 cm clear liquid above 4.5 cm resuspended powder |
| 30B | 8 | fair | 1.5 cm clear liquid above 4 cm resuspended powder |
| 31A | 4 | good | gel |
| 31B | 8 | good | very thick gel |
| 32A | 4 | no | unsuspended powder |
| 32B | 8 | no | unsuspended powder |

*All descriptions made at minimum 1 day, maximum 30 days.

TABLE X

| Additive | %$Al_2O_3$ |
|---|---|
| none | 65.31 |
| sucrose | 56.07 |
| lactose | 56.36 |
| sorbitol | 56.74 |
| propylene glycol | 63.74 |
| urea | 59.29 |
| Carbowax 600 | 56.83 |
| Carbowax 400 | 56.42 |
| Veegum | 54.40 |
| corn starch | 51.26 |
| PVP K-30 | 55.35 |
| citric acid | 57.66 |
| stearic acid | 63.66 |
| gelatin | 57.75 |
| PVP K-60 | 55.68 |
| Carbowax 200 | 54.21 |
| glycine | 58.61 |
| L-gluatmic acid | 58.72 |
| beta-alanine | 58.46 |
| potassium citrate | 56.97 |

TABLE XI

| Example No. | Additive | Wt.% $Al_2O_3$ resuspension | Drops HCl (31%) added |
|---|---|---|---|
| 34B | sucrose | 8 | 3 (0.14 g) |
| 34C | sucrose | 4 | 3 |
| 34D | sucrose | 8 | 5 (0.24 g) |
| 35A | lactose | 4 | 3 |
| 35B | lactose | 8 | 5 |
| 35C | lactose | 4 | 3 |
| 35D | lactose | 8 | 3 |
| 36A | sorbitol | 4 | 3 |
| 36B | sorbitol | 8 | 5 |
| 36C | sorbitol | 4 | 3 |
| 36D | sorbitol | 8 | 3 |
| 37A | propylene glycol | 4 | 3 |
| 37B | propylene glycol | 8 | 5 |
| 37C | propylene glycol | 4 | 3 |
| 37D | propylene glycol | 8 | 3 |
| 38A | Carbowax 200 | 4 | 3 |
| 38B | Carbowax 200 | 8 | 3 |
| 38C | Carbowax 200 | 4 | 3 |
| 38D | Carbowax 200 | 8 | 5 |
| 39A | Carbowax 400 | 4 | 3 |
| 39B | Carbowax 400 | 8 | 5 |
| 39C | Carbowax 400 | 4 | 3 |
| 39D | Carbowax 400 | 8 | 3 |
| 40A | Carbowax 600 | 4 | 3 |
| 40B | Carbowax 600 | 8 | 5 |
| 40C | Carbowax 600 | 4 | 3 |
| 40D | Carbowax 600 | 8 | 3 |
| 41A | stearic acid | 4 | 3 |
| 41B | stearic acid | 8 | 5 |
| 41C | stearic acid | 4 | 3 |
| 41D | stearic acid | 8 | 3 |
| 42A | citric acid | 4 | 5 |
| 42B | citric acid | 8 | 3 |
| 42C | citric acid | 4 | 3 |
| 42D | citric acid | 8 | 3 |
| 43A | potassium citrate | 4 | 3 |
| 43B | potassium citrate | 8 | 5 |
| 43C | potassium citrate | 8 | 3 |
| 44A | corn starch | 4 | 3 |
| 44B | corn starch | 8 | 5 |
| 44C | corn starch | 4 | 3 |
| 44D | corn starch | 8 | 3 |
| 45A | urea | 4 | 5 |
| 45B | urea | 8 | 3 |
| 45C | urea | 4 | 3 |
| 45D | urea | 8 | 3 |
| 46A | glycine | 4 | 3 |
| 46B | glycine | 8 | 5 |
| 46C | glycine | 4 | 3 |
| 46D | glycine | 8 | 3 |
| 47A | beta-alanine | 4 | 3 |
| 47B | beta-alanine | 8 | 5 |
| 47C | beta-alanine | 4 | 3 |
| 47D | beta-alanine | 8 | 3 |
| 48A | L-glutamic acid | 4 | 3 |
| 48B | L-glutamic acid | 8 | 5 |
| 48C | L-glutamic acid | 4 | 0 |
| 48D | L-glutamic acid | 8 | 3 |
| 49A | gelatin | 4 | 3 |
| 49B | gelatin | 8 | 5 |

TABLE XI (Cont'd)

| Example No. | Additive | Wt.% $Al_2O_3$ resuspension | Drops HCl (31%) added |
|---|---|---|---|
| 49C | gelatin | 8 | 3 |
| 50A | Veegum | 4 | 3 |
| 50B | Veegum | 8 | 5 |
| 50C | Veegum | 4 | 3 |
| 50D | Veegum | 8 | 3 |

TABLE XII

| Example No. | Additive | Adjusted pH | $\%Al_2O_3$ | Resuspension | *Description |
|---|---|---|---|---|---|
| 34A | sucrose | 5.2 | 4 | good | thick gel |
| 34B | sucrose | 6.7 | 8 | good | fluid gel |
| 34C | sucrose | 6.0 | 4 | good | thick gel |
| 34D | sucrose | 5.6 | 8 | good | very thick gel |
| 35A | lactose | 5.5 | 4 | good | thick gel |
| 35B | lactose | 5.6 | 8 | good | thick gel |
| 35C | lactose | 6.3 | 4 | good | thick fluid gel |
| 35D | lactose | 6.6 | 8 | good | fluid gel |
| 36A | sorbitol | 5.5 | 4 | good | gel |
| 36B | sorbitol | 6.4 | 8 | good | very thick gel |
| 36C | sorbitol | 6.3 | 4 | good | fluid gel |
| 36D | sorbitol | 6.5 | 8 | good | gel |
| 37A | propylene glycol | 5.2 | 4 | good | gel |
| 37B | propylene glycol | 6.5 | 8 | fair | 0.5 cm clear liquid on 5 cm powder |
| 37C | propylene glycol | 6.5 | 4 | no | unsuspended powder |
| 37D | propylene glycol | 6.7 | 8 | fair | 0.5 cm clear liquid on 5 cm powder |
| 38A | Carbowax 200 | 5.5 | 4 | good | thick fluid gel |
| 38B | Carbowax 200 | 6.4 | 8 | good | thick fluid gel |
| 38C | Carbowax 200 | 5.8 | 4 | good | gel |
| 38D | Carbowax 200 | 5.6 | 8 | good | gel |
| 39A | Carbowax 400 | 4.6 | 4 | good | thick fluid gel |
| 39B | Carbowax 400 | 6.1 | 8 | good | very thick gel |
| 39C | Carbowax 400 | 6.0 | 4 | good | gel |
| 39D | Carbowax 400 | 6.4 | 8 | good | thick fluid gel |
| 40A | Carbowax 600 | 4.7 | 4 | good | thick fluid gel |
| 40B | Carbowax 600 | 5.1 | 8 | good | gel |
| 40C | Carbowax 600 | 5.4 | 4 | good | thick fluid gel |
| 40D | Carbowax 600 | 6.2 | 8 | no | unsuspended powder |
| 41A | stearic acid | 4.1 | 4 | no | unsuspended powder with hazy liquid |
| 41B | stearic acid | 5.1 | 8 | no | same as above |
| 41C | stearic acid | 4.3 | 4 | no | same as above |
| 41D | stearic acid | 5.2 | 8 | no | same as above |
| 42A | citric acid | 2.5 | 4 | no | unsuspended powder |
| 42B | citric acid | 2.5 | 8 | no | same as above |
| 42C | citric acid | 2.6 | 4 | no | same as above |
| 42D | citric acid | 2.7 | 8 | no | same as above |
| 43A | potassium citrate | 5.8 | 4 | no | same as above |
| 43B | potassium citrate | 6.6 | 8 | no | same as above |
| 43C | potassium citrate | 8.3 | 8 | no | same as above |
| 44A | corn starch | 4.8 | 4 | good | fluid gel |
| 44B | corn starch | 5.1 | 8 | good | thick fluid gel |
| 44C | corn starch | 6.1 | 4 | no | unsuspended powder |
| 44D | corn starch | 6.2 | 8 | no | same as above |
| 45A | urea | 4.9 | 4 | good | fluid gel |

TABLE XII (Cont'd)

| Example No. | Additive | Adjusted pH | %$Al_2O_3$ | Resuspension | *Description |
|---|---|---|---|---|---|
| 45B | urea | 5.7 | 8 | good | very thick gel |
| 45C | urea | 5.6 | 4 | good | thick gel |
| 45D | urea | 6.5 | 8 | no | unsuspended powder |
| 46A | glycine | 5.2 | 4 | good | gel |
| 46B | glycine | 5.4 | 8 | good | gel |
| 46C | glycine | 5.6 | 4 | good | gel |
| 46D | glycine | 6.6 | 8 | fair | 1 cm clear liquid above 4.5 cm powder |
| 47A | beta-alanine | 5.0 | 4 | good | fluid gel |
| 47B | beta-alanine | 6.0 | 8 | good | gel |
| 47C | beta-alanine | 6.2 | 4 | good | gel |
| 47D | beta-alanine | 6.7 | 8 | fair | 0.5 cm clear liquid above powder |
| 48A | L-glutamic acid | 3.7 | 4 | good | gel |
| 48B | L-glutamic acid | 3.9 | 8 | good | thick fluid gel |
| 48C | L-glutamic | 4.9 | 4 | no | unsuspended powder |
| 48D | L-glutamic | 4.1 | 8 | good | thick fluid gel |
| 49A | gelatin | 5.3 | 4 | no | same as above |
| 49B | gelatin | 6.1 | 8 | no | same as above |
| 49C | gelatin | 6.6 | 8 | no | same as above |
| 50A | Veegum | 5.5 | 4 | no | same as above |
| 50B | Veegum | 6.3 | 8 | no | same as above |
| 50C | Veegum | 6.6 | 4 | no | same as above |
| 50D | Veegum | 6.7 | 8 | no | same as above |

TABLE XIII

Protein binding capacity of $Al_2O_3$ with additives*

| Example No. | Sample description | %$Al_2O_3$ | mg. of Protein adsorbed / mg. of $Al_2O_3$ |
|---|---|---|---|
| 51 | $Al_2O_3$ slurry (no additive) | 3.37 | 1.34 |
| 52 | $Al_2O_3$ w/L-glutamic acid | 58.72 | 0.48 |
| 53 | $Al_2O_3$ w/Carbowax-600 | 56.83 | 0.25 |
| 54 | $Al_2O_3$ w/beta-alanine | 58.46 | 0.24 |
| 55 | $Al_2O_3$ w/Carbowax-400 | 56.42 | 0.21 |
| 56 | $Al_2O_3$ w/stearic acid | 63.62 | 0.20 |
| 57 | $Al_2O_3$ w/K-citrate | 56.92 | 0.20 |
| 58 | $Al_2O_3$ w/citric acid | 57.66 | 0.17 |
| 59 | $Al_2O_3$ w/Glycine | 58.61 | 0.17 |
| 60 | $Al_2O_3$ w/propylene glycol | 63.74 | 0.14 |
| 61 | $Al_2O_3$ w/urea | 59.29 | 0.13 |
| 62 | $Al_2O_3$ w/sucrose | 56.07 | 0.12 |
| 63 | $Al_2O_3$ w/Carbowax-200 | 59.21 | 0.12 |
| 64 | $Al_2O_3$ (no additive) | 65.31 | 0.11 |
| 65 | $Al_2O_3$ w/sorbitol | 56.74 | 0.11 |
| 66 | $Al_2O_3$ w/gelatin | 57.75 | 0.10 |
| 67 | $Al_2O_3$ w/PVP (K-60) | 55.68 | 0.09 |
| 68 | $Al_2O_3$ w/PVP (K-30) | 55.35 | 0.08 |
| 69 | $Al_2O_3$ w/lactose | 56.36 | 0.04 |
| 70 | $Al_2O_3$ w/Veegum | 54.40 | 0.0 |
| 71 | $Al_2O_3$ w/corn starch | 51.26 | 0.0 |

*$Al_2O_3$ powder with 3:1 additive ratio

TABLE XIV

| Example No. | Co-dried additive | % $Al_2O_3$ | % Additive | Aprox. $Al_2O_3$ | Actual $Al_2O_3$ | Nat'l pH | Protein binding: PB @ nat'l pH (mg Protein / Mg $Al_2O_3$) | Protein binding: PB @* pH 5.51 (mg Protein / Mg $Al_2O_3$) |
|---|---|---|---|---|---|---|---|---|
| 72 | None | 65.3 | 0 | — | — | 8.6 | 0.11 | 0 |
| 73 | Glycerine | 61.4 | 10.75 | 6/1 | 5.7/1 | | 0.09 | 0.54 |
| 74 | Glycerine | 60.1 | 21.5 | 3/1 | 2.8/1 | 8.5 | 0.11 | 0.95 |
| 75 | Glycerine | 57.9 | 43 | 1.5/1 | 1.3/1 | | 0.26 | 1.25 |
| 76 | Glycerine/HCl | | | 3/1 | | 5.5 | 1.7 | 1.7 |
| 77 | Glycerine/HCl | 62.1 | 15.7 | | 4.0/1 | 4.7 | | 1.7 |
| 78 | None | 65.3 | 0 | — | — | 8.6 | 0.11 | 0 |
| 79 | Glycerine | 60.1 | 21.6 | 3/1 | 2.8/1 | 8.5 | 0.11 | 0.95 |
| 80 | Carbowax 400 | 56.4 | | 3/1 | | 8.1 | 0.21 | 0.73 |
| 81 | Lactose | 56.4 | | 3/1 | | 8.8, 8.7 | 0.04 | 0.67 |
| 82 | Glycine | 58.6 | | 3/1 | | 8.1 | 0.17 | 0.58 |
| 83 | Sorbitol | 56.7 | | 3/1 | | 8.8, 8.7 | 0.11 | 0.36 |
| 84 | Propylene glycol | 63.7 | | 3/1 | | 8.7, 8.6 | 0.14 | 0.34 |
| 85 | Citric acid | 57.7 | | 3/1 | | 3.4 | 0.17 | 0.08 |
| 86 | Stearic acid | 63.6 | | 3/1 | | 7.8 | 0.20 | 0.05 |
| 87 | Corn starch | 51.3 | | 3/1 | | 8.2 | 0 | 0 |
| 88 | Gelatin | 57.8 | | 3/1 | | 8.0 | 0.10 | 0 |
| 89 | K citrate | 57.0 | | 3/1 | | 9.4 | 0.20 | 0.1 |
| 90 | Arlacel 165** | 58.0 | | 3/1 | | 8.2 | | 0 |
| 91 | Arlacel 165** | 55.9 | | 3/1 | | | | 0.17 |

*Adjusted with HCl or caustic to yield pH 5.51
**Arlacel 165—95% glycerol monostearate and 5% polyethylene glycol monostearate

Examples 92—100

TABLE XV
Co-dried $Al_2O_3$ and glycerol—with and without HCl

| Example No. | HCl | $Al_2O_3$/glycerol ratio in slurry | %$Al_2O_3$ in powder | Protein binding mg protein/ mg $Al_2O_3$ | pH of 4% $Al_2O_3$ suspension | Resuspend-ability |
|---|---|---|---|---|---|---|
| 92 | No HCl | 3/1 | 58.9 | 0.04 | 9.0 | Poor |
| 93 | No HCl | 3/0.5 | 61.4 | 0.09 | 8.5 | Poor |
| 94 | No HCl | 3/1 | 60.1 | 0.11 | 8.5 | Poor |
| 95 | No HCl | 3/1.5 | 58.6 | 0.15 | 8.5 | Poor |
| 96 | No HCl | 3/2 | 57.9 | 0.26 | 8.5 | Poor |
| 97 | No HCl | 3/1 | 62.48 | 0.4 | 8.5 | Poor |
| 98 | HCl | 3/1 | 62.48 | 1.7 | 4.7* | Good |
| 99 | HCl | 3/1 | — | 1.6 | * | Good |
| 100 | HCl | 3/1 | 58.13 | 1.78 | 6.2 | Good |

*pH was adjusted to 5.5 before protein binding test

## Claims

1. A method for the preparation of adsorptive, protein-binding aluminium oxyhydroxide, which comprises mixing an aqueous suspension of a carbonate-free, highly hydrated, aluminium oxyhydroxide gel which is relatively inert towards weak alkalis and acids, with a water-soluble additive which is a polyhydric alcohol, sugar, amino acid, polysaccharide or polyfunctional nitrogen-containing compound and which is capable of providing coordinating moieties with aluminium, and drying the mixture to form solid particles.

2. A method according to claim 1, in which the additive is added in such amount that the weight ratio of aluminium oxyhydroxide, calculated as aluminium oxide, to additive in the suspension before drying, is from 10:1 to 3:2.

3. A method according to claim 1 or 2, in which the mixture is dried such that the proportion of aluminium oxyhydroxide, calculated as aluminium oxide, in the product is from 55 to 65% by weight.

4. A method according to any of claims 1 to 3, which further comprises mixing water with the dried aluminium oxyhydroxide and acidifying the resulting mixture to a pH of from 4 to 6.5 to produce a resuspendable, adsorptive, protein-binding aluminium oxyhydroxide.

5. A method according to claim 4, in which the pH is from 5 to 6.

6. A method according to any of claims 1 to 3, in which, before drying and either before or after mixing the aluminium oxyhydroxide suspension with the additive, the suspension is acidified to a pH of from 4 to 6.5 to produce a re-suspendable, adsorptive, protein-binding aluminium oxyhydroxide.

7. A method according to claim 6, in which the pH is from 5 to 6.

8. A method according to claims 6 or 7, which further comprises mixing water with the dried aluminium oxyhydroxide to produce a resuspended, adsorptive, protein-binding aluminium oxyhydroxide.

9. A composition for topical application to the skin which comprises or consists of adsorptive, protein-binding aluminium oxyhydroxide produced by a method according to any of claims 1 to 3.

## Patentansprüche

1. Verfahren zur Herstellung von adsorbierendem proteinbindendem Aluminiumoxyhydroxid umfassend das Vermischen einer wäßrigen Suspension carbonatfreien, hochhydratisierten Aluminiumoxyhydroxidgels, welches gegen schwache Alkalien und Säuren vergleichsweise inert ist, mit einem wasserlöslichen Additiv in Form eines mehrwertigen Alkohols, eines Zuckers, einer Aminosäure, eines Polysaccharids oder einer polyfunktionellen, Stickstoff enthaltenden Verbindung, und welches in der Lage ist, Koordinationsstellen mit Aluminium bereitzustellen, und Trocknen des Gemischs unter Bildung fester Teilchen.

2. Verfahren gemäß Anspruch 1, wobei das Additiv in solch einer Menge zugefügt wird, daß das Gewichtsverhältnis Aluminiumoxyhydroxid, berechnet als Aluminiumoxid, zu Additiv in der Suspension vor dem Trocknen zwischen 10:1 und 3:2 beträgt.

3. Verfahren gemäß Anspruch 1 oder 2, wobei das Gemisch so getrocknet wird, daß der Anteil an Aluminiumoxyhydroxid, berechnet als Aluminiumoxid, im Produkt zwischen 55 und 65 Gew.-% beträgt.

4. Verfahren gemäß einem der Ansprüche 1 bis 3, weiter umfassend das Vermischen von Wasser mit dem getrockneten Aluminiumoxyhydroxid und das Ansäuern des erhaltenen Gemischs auf einen pH zwischen 4 und 6,5 unter Bildung eines wiedersuspendierbaren, adsorbierenden, proteinbindenden Aluminiumoxyhydroxids.

5. Verfahren gemäß Anspruch 4, wobei der pH zwischen 5 und 6 beträgt.

6. Verfahren gemäß einem der Ansprüche 1 bis 3, wobei die Suspension unter Bildung eines wiedersuspendierbaren, adsorbierenden, proteinbindenden Aluminiumoxyhydroxids vor dem Trocknen und entweder vor oder nach dem Vermischen der Aluminiumoxyhydroxidsuspension mit dem Additiv auf einen pH zwischen 4 und 6,5 angesäuert wird.

7. Verfahren gemäß Anspruch 6, wobei der pH 5 bis 6 beträgt.

8. Verfahren gemäß Anspruch 6 oder 7 weiter umfassend das Vermischen von Wasser mit dem getrockneten Aluminiumoxyhydroxid unter Bildung eines wiedersuspendierten, adsorbierenden, proteinbindenden Aluminiumoxyhydroxids.

9. Zusammensetzung zur topischen Anwendung auf der Haut umfassend oder bestehend aus adsorbierendem, proteinbindendem Aluminiumoxyhydroxid, welches nach einem Verfahren gemäß einem der Ansprüche 1 bis 3 hergestellt wurde.

## Revendications

1. Procédé de préparation d'oxyhydroxyde d'aluminium adsorbant, fixant les protéines, qui comprend le mélange d'une suspension aqueuse d'un gel d'oxyhydroxyde d'aluminium hautement hydraté, sans carbonate, qui est relativement inerte vis-à-vis des bases et acides faibles, avec un additif hydrosoluble qui est un polyalcool, un sucre, un acide aminé, un polysaccharide ou un composé azoté polyfonctionnel et qui est capable de donner des groupements de coordination avec l'aluminium, et le séchage du mélange pour former des particules solides.

2. Procédé selon la revendication 1, dans lequel l'additif est ajouté en une quantité telle que le rapport pondéral de l'oxyhydroxyde d'aluminium, calculé en oxyde d'aluminium, à l'additif, dans la suspension avant le séchage, est de 10:1 à 3:2.

3. Procédé selon la revendication 1 ou 2, dans lequel le mélange est séché de telle manière que la proportion d'oxyhydroxyde d'aluminium, calculée en oxyde d'aluminium, dans le produit soit de 55 à 65% en poids.

4. Procédé selon l'une quelconque des revendications 1 à 3, qui comprend en outre le mélange d'eau avec l'oxyhydroxyde d'aluminium séché et l'acidification du mélange résultant jusqu'à un pH de 4 à 6,5 pour produire un oxyhydroxyde d'aluminium adsorbant, fixant les protéines, pouvant être remis en suspension.

5. Procédé selon la revendication 4, dans lequel le pH est de 5 à 6.

6. Procédé selon l'une quelconque des revendications 1 à 3, dans lequel, avant le séchage et soit avant soit après le mélange de la suspension d'oxyhydroxyde d'aluminium avec l'additif, la suspension est acidifiée jusqu'à un pH de 4 à 6,5 pour produire un oxyhydroxyde d'aluminium adsorbant, fixant les protéines, pouvant être remis en suspension.

7. Procédé selon la revendication 6, dans lequel le pH est de 5 à 6.

8. Procédé selon la revendication 6 ou 7, qui comprend en outre le mélange d'eau avec l'oxyhydroxyde d'aluminium séché pour produire un oxyhydroxyde d'aluminium adsorbant, fixant les protéines, pouvant être remis en suspension.

9. Composition pour application topique sur la peau qui comprend ou qui consiste en un oxyhydroxyde d'aluminium adsorbant, fixant les protéines, produit par un procédé selon l'une quelconque des revendications 1 à 3.

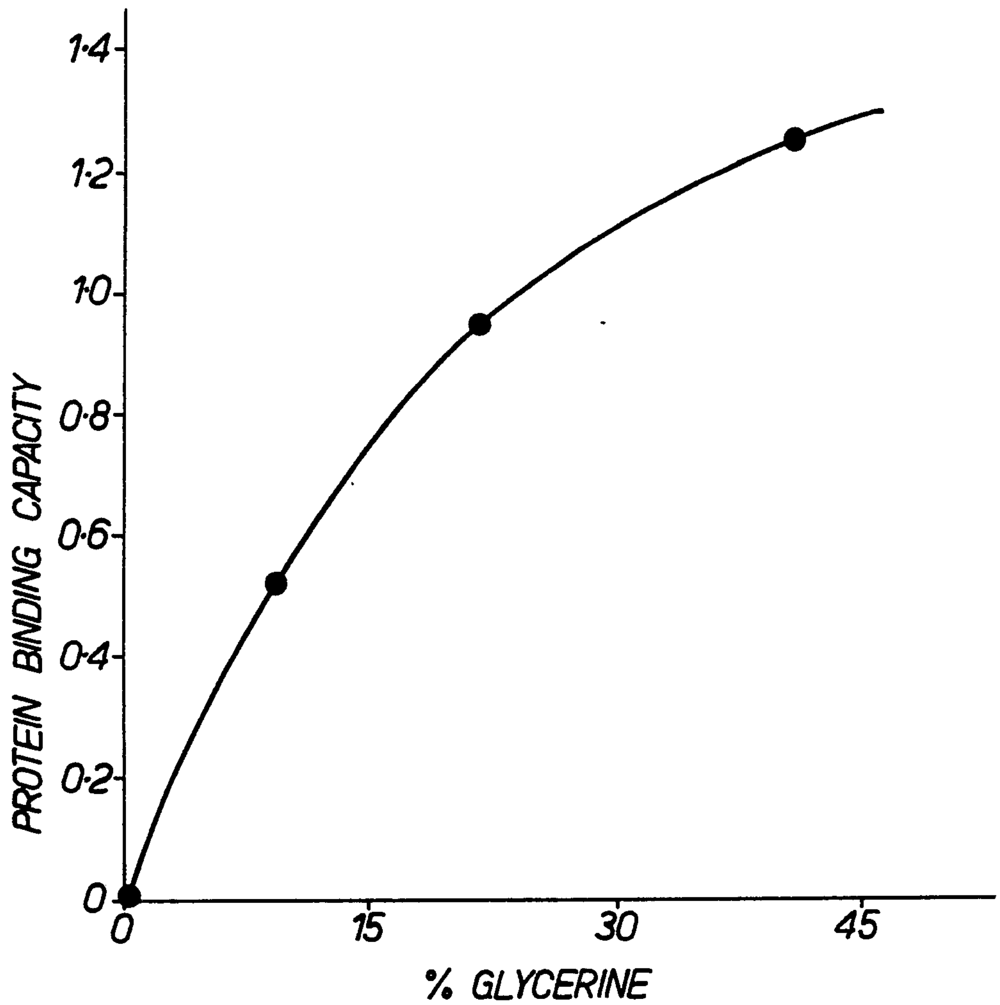
1·4
1·2
1·0
0·8
0·6
0·4
0·2
0
PROTEIN BINDING CAPACITY
0
15
30
45
% GLYCERINE